(19) Europäisches Patentamt
European Patent Office
Office européen des brevets

(11) Veröffentlichungsnummer: **0 050 253**
**A1**

(12) **EUROPÄISCHE PATENTANMELDUNG**

(21) Anmeldenummer: **81107891.4**

(22) Anmeldetag: **03.10.81**

(51) Int. Cl.³: **A 61 K 31/43,** A 61 K 31/545 // (A61K31/43, 31/42),(A61K31/545, 31/42)

(30) Priorität: **17.10.80 DE 3039243**

(43) Veröffentlichungstag der Anmeldung: **28.04.82** ***Patentblatt 82/17***

(84) Benannte Vertragsstaaten: **BE CH DE FR GB IT LI LU NL SE**

(71) Anmelder: **Dr. Karl Thomae GmbH, Postfach 1755, D-7950 Biberach (Riss) (DE)**

(72) Erfinder: **Wetzel, Bernd, Dr., Dipl.-Chem., Kapellenweg 21, D-7950 Biberach 1 (DE)**
Erfinder: **Lechner, Uwe, Dr., Panoramastrasse 12, D-7951 Ummendorf (DE)**

(54) **Antibiotisch wirksame Arzneimittelkombination.**

(57) Antibiotisch wirksame Arzneimittelkombination, bestehend aus einer Mischung von D-α-[3-(2-p-Aminosulfonylanilino-4-hydroxy-5-pyrimidinyl)-ureido]-p-hydroxy-benzyl-penicillansäure und/oder 7-{D-α-[3-(2-p-Aminosulfonylanilino-4-hydroxy-5-pyrimidinyl)-ureido]-p-hydroxyphenyl-acetamido}-3-[(1-methyl-tetrazol-5-yl)-thiomethyl]-ceph-3-em-4-carbonsäure
und/oder deren pharmakologisch verträglichen Salzen mit anorganischen oder organischen Basen mit Z-(2R,5R)-3-(β-Hydroxyäthyliden)-7-oxo-4-oxy-1-azabicyclo[3,2,0]-heptan-2-carvonsäure der Formel

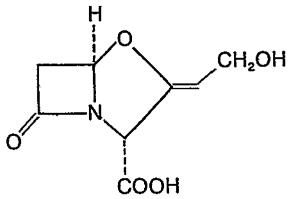

und/oder mit deren pharmakologisch verträglichen Salzen mit anorganischen oder organischen Basen.

EP 0 050 253 A1

Case 5/805
Dr.Bu/fra

DR. KARL THOMAE GMBH, BIBERACH AN DER RISS

Antibiotisch wirksame Arzneimittelkombination

Die Erfindung betrifft eine neue Mischung eines ß-Lactam-antibiotikums und einer ß-Lactamase inhibierenden Verbindung und ein eine solche Mischung enhaltendes Arzneimittel.

Als ß-Lactam-Antibiotikum dienen die D-α-[3-(2-p-Aminosulfonylanilino-4-hydroxy-5-pyrimidinyl)-ureido]-p-hydroxy-benzylpenicillansäure und/oder die 7-{D-α-[3-(2-p-Aminosulfonylanilino-4-hydroxy-5-pyrimidinyl)-ureido]-p-hydroxy-phenylacetamido}-3-[(1-methyl-tetrazol-5-yl)-thiomethyl]-ceph-3-em-4-carbonsäure oder deren pharmakologisch verträgliche Salze mit anorganischen oder organischen Basen, als ß-Lactamase-Inhibitor dient Z-(2R+5R)-3-(ß-Hydroxyäthyliden)-7-oxo-4-oxa-1-azabicyclo [3,2,0]-heptan-2-carbonsäure der Formel

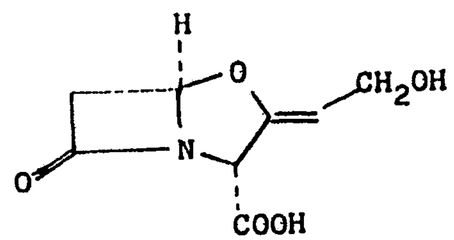

sowie deren pharmakologisch verträgliche Salze mit anorganischen oder organischen Basen.

Bei den obenerwähnten pharmakologisch verträglichen Salzen handelt es sich um allgemein verwendete Penicillin- oder Cephalosporinsalze, insbesondere um das Natrium- oder Kaliumsalz, oder um Salze mit Aminen, wie N,N-Dibenzyläthylendiamin.

Es wurde gefunden, daß ß-Lactamasen bei Vorliegen der obenerwähnten Clavulansäure bzw. ihrer Salze nur sehr erschwert die obengenannte Penicillansäure oder Ceph-3-em-4-carbonsäure oder deren Salze angreift; überraschenderweise hat sich dabei gezeigt, daß die beiden Mischungskomponenten im Sinne eines synergistischen Effektes verstärkt antibakteriell wirken.

Das erfindungsgemäße Gemisch bzw. Mittel enthält das ß-Lactam-Antibiotikum und die Clavulansäure bzw. ihre Salze vorzugsweise in einem Gewichtsverhältnis von 10:1 bis 1:10, insbesondere aber in einem Verhältnis von 5:1 bis 1:3.

Je nach der Art der zu bekämpfenden Bakterien kann entweder die obengenannte Penicillansäure oder die obengenannte Ceph-3-em-4-carbonsäure bzw. ihre Salze oder auch ein Gemisch dieser Säuren bzw. ihrer Salze verwendet werden.

Die obengenannte Penicillansäure wird in der DE-OS 28 08 153, die Ceph-3-em-4-carbonsäure in der DE-OS 29 24 948 beschrieben. Diese Verbindungen sind stark antibakteriell wirksam, sie zeigen insbesondere eine sehr gute Wirkung gegen gramnegative Bakterien.

Die Aufgabe vorliegender Erfindung besteht in der Schaffung eines antibakteriellen Gemisches bzw. eines ein solches Gemisch enthaltenden Arzneimittels, das ganz besonders gut gegen gramnegative Bakterien, beispielsweise gegen Klebsiella pneumoniae, Pseudomonas aeruginosa, Escherichia coli und verschiedene Proteus-Keime wirkt und dabei auch noch eine gute Wirkung gegen grampositive Keime zeigt, wobei die Wirkung auch gegen Keime, die gegenüber den üblichen Penicillinen und Cephalosporinen resistent sind, voll erhalten bleibt. Eine weitere Aufgabe der Erfindung besteht in der Schaffung eines Gemisches, bei welchem die antibakterielle Aktivität der einen Komponenten durch eine andere Komponente gesteigert wird.

Das erfindungsgemäße antibakterielle Mittel wird vorzugsweise parenteral appliziert. Es kann auch in Form einer Salbe oder eines Puders oder in Form eines Präparates für die rektale Applikation vorliegen.

Zum Zwecke der Injektion wird das Gemisch in geeigneten festen oder flüssigen Trägerstoffen oder Verdünnungsmitteln vermischt; als Trägerstoff kommt beispielsweise sterilisiertes Wasser in Frage. Feste Trägerstoffe dienen beispielsweise zur Herstellung eines Arzneimittels, welches vor der Applikation in sterilisiertem Wasser oder in einer Glucoselösung oder in einer physiologischen Kochsalzlösung gelöst wird. Für Injektionslösungen verwendet man vorzugsweise eine Dosierung von 0,5 bis 10 g des Gemisches/Tag für Erwachsene; die Dosierung hängt von der Art der Infektion und vom Patienten ab und kann in weiten Grenzen variiert werden.

Die folgenden Testversuche sollen die Wirksamkeit des antibakteriellen, erfindungsgemäßen Mittels zeigen.

Ein Caseinpepton-Fleischextrakt-Bouillon-Agar mit einem Gehalt der angegebenen Menge der nachstehend angegebenen Substanzen A und B wird mit dem Testbakterium geimpft (~ $3x10^4$ CFU/ml). Nach der Inkubation während 18 Stunden bei 37°C wird der Wuchszustand der Testbakterien untersucht. Die Ergebnisse sind in den Tabellen zusammengestellt. In jeder Tabelle bedeutet (+), daß das Testbakterium wächst, und (-) bedeutet, daß das Testbakterium nicht wächst. Man erkennt aus diesen Tabellen, daß die Kombination von

D-α-[3-(2-p-Aminosulfonylanilino-4-hydroxy-5-pyrimidinyl)-ureido]-p-hydroxy-benzylpenicillin-Natrium = A

bzw.

Natrium-7-{D-α-[3-(2-p-aminosulfonylanilino-4-hydroxy-5-pyrimidinyl)-ureido]-p-hydroxy-phenylacetamido}-3-[(1-methyl-tetrazol-5-yl)-thiomethyl]-ceph-3-em-4-carboxylat = B

mit

Lithium-Z-(2R,5R)-3-(ß-hydroxyäthyliden)-7-oxo-4-oxa-1-aza-bicyclo[3,2,0]-heptan-2-carboxylat = C

einen synergistischen Effekt hinsichtlich der Inhibierung des Wachstums der pathogenen Bakterien liefert.

Tabelle 1

Substanz C µg/ml — Escherichia coli $R^+$TEM

| | | | | | | | | | | | |
|---|---|---|---|---|---|---|---|---|---|---|---|
| 32 | - | - | - | - | - | - | - | - | - | - | |
| 16 | - | - | - | - | - | - | - | - | - | + | |
| 8 | - | - | - | - | - | - | - | - | - | + | |
| 4 | - | - | - | - | - | - | - | - | - | + | |
| 2 | - | - | - | - | - | - | - | - | + | + | |
| 1 | - | - | - | - | - | - | - | + | + | + | |
| 0 | + | + | + | + | + | + | + | + | + | + | |
| | 128 | 64 | 32 | 16 | 8 | 4 | 2 | 1 | 0,5 | 0 | Substanz A µg/ml |

Tabelle 2

Substanz C µg/ml — Kl. pneumoniae 1082 E

| | | | | | | | | | | | |
|---|---|---|---|---|---|---|---|---|---|---|---|
| 64 | - | - | - | - | - | - | - | - | - | - | |
| 32 | - | - | - | - | - | - | - | - | + | + | |
| 16 | - | - | - | - | - | - | + | + | + | + | |
| 8 | - | - | - | - | - | + | + | + | + | + | |
| 4 | - | - | - | - | + | + | + | + | + | + | |
| 2 | - | - | - | + | + | + | + | + | + | + | |
| 1 | - | + | + | + | + | + | + | + | + | + | |
| 0 | + | + | + | + | + | + | + | + | + | + | |
| | 64 | 32 | 16 | 8 | 4 | 2 | 1 | 0,5 | 0,25 | 0 | Substanz A µg/ml |

Tabelle 3

Substanz C µg/ml E. coli $R^{+}$TEM

| | | | | | | | | | |
|---|---|---|---|---|---|---|---|---|---|
| 32 | - | - | - | - | - | - | - | - | - |
| 16 | - | - | - | - | - | - | - | - | + |
| 8 | - | - | - | - | - | - | - | - | + |
| 4 | - | - | - | - | - | - | - | - | + |
| 2 | - | - | - | - | - | - | - | - | + |
| 1 | - | - | - | - | - | - | - | - | + |
| 0,5 | - | - | - | - | - | - | - | + | + |
| 0,25 | - | - | - | - | - | - | + | + | + |
| 0 | - | + | + | + | + | + | + | + | + |
| | 8 | 4 | 2 | 1 | 0,5 | 0,25 | 0,125 | 0,06 | 0 |

Substanz B µg/ml

Tabelle 4

Substanz C µg/ml Kl. pneumoniae 1082 E

| | | | | | | | | | | |
|---|---|---|---|---|---|---|---|---|---|---|
| 64 | - | - | - | - | - | - | - | - | - | - |
| 32 | - | - | - | - | - | - | - | - | - | + |
| 16 | - | - | - | - | - | - | - | - | + | + |
| 8 | - | - | - | - | - | - | - | + | + | + |
| 4 | - | - | - | - | - | - | + | + | + | + |
| 2 | - | - | - | - | - | + | + | + | + | + |
| 1 | - | - | + | + | + | + | + | + | + | + |
| 0 | + | + | + | + | + | + | + | + | + | + |
| | 64 | 32 | 16 | 8 | 4 | 2 | 1 | 0,5 | 0,25 | 0 |

Substanz B µg/ml

Die in den vorstehenden Testversuchen 1 bis 4 erhaltenen Ergebnisse sind typisch für die pharmakologische Aktivität des antibakteriellen Mittels gemäß vorliegender Erfindung.

Man erkennt aus vorstehender Beschreibung, daß das erfindungsgemäße antibakterielle Mittel sich nicht nur ausgezeichnet zur Therapie verschiedenster Krankheiten eignet, welche durch Bakterien hervorgerufen werden, die gegenüber dem genannten Penicillin und Cephalosporin allein empfindlich sind, sondern auch hervorragend geeignet ist zur Behandlung von Infektionskrankheiten, die durch ß-Lactamasetragende Bakterien hervorgerufen werden und bei denen das genannte Penicillin oder Cephalosporin allein unter Umständen zu keinem Therapieerfolg führt.

Im folgenden wird die Erfindung anhand von Beispielen näher erläutert.

Beispiel 1

| | |
|---|---|
| Sterilisiertes D-α-[3-(2-p-Aminosulfonylanilino-4-hydroxy-5-pyrimidinyl)-ureido]-p-hydroxy-benzylpenicillin-Natrium | 500 mg |
| Sterilisiertes Li-clavulanat | 500 mg |

Die obigen Komponenten werden in 20 ml einer physiologischen Kochsalzlösung aufgelöst, wobei man eine injizierbare Lösung erhält.

Beispiel 2

| | |
|---|---|
| Sterilisiertes D-α-[3-(2-p-Aminosulfonylanilino-4-hydroxy-5-pyrimidinyl)-ureido]-p-hydroxy-benzylpenicillin-Natrium | 1,0 g |
| Sterilisiertes Li-clavulanat | 500 mg |

Die obigen Komponenten werden in 20 ml einer physiologischen Kochsalzlösung aufgelöst, wobei man eine injizierbare Lösung erhält.

Beispiel 3

Sterilisiertes Natrium-7-{D-α-/3-(2-p-aminosulfonylanilino-4-hydroxy-5-pyrimidinyl)-ureido7-p-hydroxy-phenylacetamido}-3-/(1-methyl-tetrazol-5-yl)-thiomethyl7-ceph-3-em-4-carboxylat 1000 mg

Sterilisiertes Lithium-clavulanat 500 mg

Die obigen Komponenten werden in 20 ml einer physiologischen Kochsalzlösung aufgelöst, wobei man eine injizierbare Lösung erhält.

Beispiel 4

Sterilisiertes Natrium-7-{D-α-/3-(2-p-aminosulfonylanilino-4-hydroxy-5-pyrimidinyl)-ureido7-p-hydroxy-phenylacetamido}-3-/(1-methyl-tetrazol-5-yl)-thiomethyl7-ceph-3-em-4-carboxylat 500 mg

Sterilisiertes Lithium-clavulanat 500 mg

Die obigen Komponenten werden in 20 ml einer physiologischen Kochsalzlösung aufgelöst, wobei man eine injizierbare Lösung erhält.

**P a t e n t a n s p r ü c h e**

1) Mischung von D-α-[3-(2-p-Aminosulfonylanilino-4-hydroxy-5-pyrimidinyl)-ureido]-p-hydroxy-benzylpenicillansäure und/oder 7-{D-α-[3-(2-p-Aminosulfonylanilino-4-hydroxy-5-pyrimidinyl)-ureido]-p-hydroxyphenylacetamido}-3-[(1-methyl-tetrazol-5-yl)-thiomethyl]-ceph-3-em-4-carbonsäure und/oder deren pharmakologisch verträglichen Salzen mit anorganischen oder organischen Basen mit Z-(2R,5R)-3-(ß-Hydroxyäthyliden)-7-oxo-4-oxa-1-azabicyclo[3,2,0]-heptan-2-carbonsäure der Formel

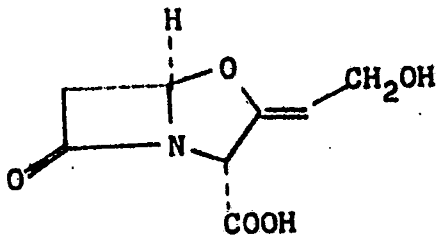

und/oder mit deren pharmakologisch verträglichen Salzen mit anorganischen oder organischen Basen.

2) Mischung gemäß Anspruch 1, bestehend aus D-α-[3-(2-p-Aminosulfonylanilino-4-hydroxy-5-pyrimidinyl)-ureido]-p-hydroxy-benzylpenicillansäure-Natrium und Lithium-Z-(2R,5R)-3-(ß-hydroxyäthyliden)-7-oxo-4-oxa-1-azabicyclo[3,2,0]-heptan-2-carboxylat.

3) Mischung gemäß Anspruch 1, bestehend aus Natrium-7-{D-α-[3-(2-p-aminosulfonylanilino-4-hydroxy-5-pyrimidinyl)-ureido]-p-hydroxy-phenylacetamido}-3-[(1-methyl-tetrazol-5-yl)-thiomethyl]-ceph-3-em-4-carboxylat und

Lithium-Z-(2R,5R)-3-(ß-hydroxyäthyliden)-7-oxo-4-oxa-1-azabicyclo/3,2,0/-heptan-2-carboxylat.

4) Mischung nach einem der Ansprüche 1 bis 3, dadurch gekennzeichnet, daß das Mischungsverhältnis (ausgedrückt als Gewichtsverhältnis oder Potenzverhältnis) des ß-Lactam-Antibiotikums und der Clavulansäure bzw. ihrer pharmakologisch verträglicher Salze im Bereich von 10:1 bis 1:10 liegt.

5) Mischung gemäß Anspruch 1, dadurch gekennzeichnet, daß diese als Komponente Lithium-Z-(2R,5R)-3-(ß-hydroxyäthyliden)-7-oxo-4-oxa-1-azabicyclo/3,2,0/-heptan-2-carboxylat enthält.

6) Mischung gemäß Anspruch 1, dadurch gekennzeichnet, daß diese als Komponente Kalium-Z-(2R,5R)-3-(ß-hydroxyäthyliden)-7-oxo-4-oxa-1-azabicyclo/3,2,0/-heptan-2-carboxylat enthält.

7) Antibakterielles Arzneimittel gekennzeichnet durch einen Gehalt eines Mittels gemäß den Ansprüchen 1 bis 4 neben den üblichen Träger- und/oder Hilfsstoffen.

0050253

Europäisches Patentamt

# EUROPÄISCHER RECHERCHENBERICHT

Nummer der Anmeldung
EP 81 10 7891

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| | EP - A - 0 000 526 (BAYER)<br>* Ansprüche *<br>--- | 1,7 | A 61 K 31/43<br>31/545//<br>A 61 K 31/43<br>31/42)<br>A 61 K 31/545<br>31/42) |
| | GB - A - 1 562 902 (BEECHAM)<br>* Ansprüche *<br>--- | 1,7 | |
| | EP - A - 0 003 814 (KARL THOMAE)<br>* Zusammenfassung; Seite 50, Absatz 2; Seiten 173,174; Beispiel 180 * | 1,2,<br>4-7 | RECHERCHIERTE SACHGEBIETE (Int. Cl.3)<br>A 61 K 31/43<br>31/545<br>31/42<br>C 07 D 499/68<br>501/36<br>498/04 |
| D | & DE - A - 2 808 153<br>---<br>---------- | | KATEGORIE DER GENANNTEN DOKUMENTE<br>X: von besonderer Bedeutung allein betrachtet<br>Y: von besonderer Bedeutung in Verbindung mit einer anderen Veröffentlichung derselben Kategorie<br>A: technologischer Hintergrund<br>O: nichtschriftliche Offenbarung<br>P: Zwischenliteratur<br>T: der Erfindung zugrunde liegende Theorien oder Grundsätze<br>E: älteres Patentdokument, das jedoch erst am oder nach dem Anmeldedatum veröffentlicht worden ist<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 06.01.1982 | CHOULY |

EPA form 1503.1 06.78